# EUROPEAN PATENT SPECIFICATION

(11) **EP 3 804 665 B1**
(45) Date of publication and mention of the grant of the patent: **29.06.2022**
(21) Application number: 20200738.1
(22) Date of filing: 08.10.2020
(51) Int. Cl.: A61F 5/02

(54) **POSTURE SHIRT**
TEXTILKORSETT ZUR VERBESSERUNG DER KÖRPERHALTUNG
CORSET TEXTILE POUR AMÉLIORER LA POSTURE

(30) Priority: 11.10.2019 IT 201900018596
(43) Date of publication of application: 14.04.2021
(73) Proprietor: F.G.P. S.R.L., 37062 Villafranca Di Verona (Verona) (IT)
(72) Inventor: FERRIGOLO, Moreno, 37062 Villafranca di Verona (IT); TURRINI, Alberto, 37062 Villafranca di Verona (IT)
(74) Representative: Sandri, Sandro

(56) References cited:
- US-A1- 2003 130 603
- US-A1- 2017 354 530
- US-A1- 2018 318 693
- US-A1- 2019 246 709

## Description

### FIELD OF APPLICATION

The present invention relates to an item of clothing of a technical-orthopaedic type represented by a shirt provided with vertebral reinforcement, produced with a technique that brings about an improvement in the posture of the person who wears it.

The present solution envisages producing a shirt both as an integral item of clothing and as a combination of several parts, the shirt comprising areas with differentiated compression or traction that determine a control of posture and further envisages the introduction, in a special back pocket, of a vertebral reinforcement that brings about a further improvement in posture.

The technology for producing the shirt provided with vertebral reinforcement according to the invention allows this item of clothing to be designed in a personalised manner, so as to obtain differentiated tensions, according to the zones, and to ensure stimulation of the muscles responsible for postural control, with the aim of restoring a correct attitude in subjects affected by hyperkyphotic dysmorphisms and the like.

The present invention has application in the sector of clothing, in particular of a medical and orthopaedic type, and in particular in the production of braces in general, as well as prostheses, and of braces mainly utilisable in conservative, post-traumatic, rehabilitative and postoperative therapy.

### PRIOR ART

In the orthopaedic-health sector, there are many known solutions envisaging the use of orthopaedic braces or orthoses of the corset type which are used in the presence of certain pathologies or in the event of some problems of an orthopaedic type affecting the spinal column or torso of persons subject to osteoporosis or other pathological forms, both degenerative and inflammatory, or also of traumatic origin. An example is to be found in US 2017/354530 A1

Traditional orthopaedic braces or orthoses of the corset type, which ensure a certain support for the patient by absorbing the most intense stresses that develop in the torso, prove to be particularly useful in cases of back pain and in the less acute stages of osteoporosis, since, through activation of the dorsal muscles, they favour a straightening of the chest with a decrease in the kyphosis caused by osteoporosis.

Various types of corsets, braces or orthoses designed to support or contain the torso are known and widely available.

These are generally structures that rest against the spinal column and consist for the most part of a plate for stiffening the spinal column, and thus shaped with the same conformation; the plate is attached by using special straps so as to be held in place on the torso.

Such structures normally comprise a rigid, vertically elongate frame made of a metallic or in any case rigid material, suitably coated, and designed according to a shape adapted to adhere to the spinal column, wherein said frame must be secured to the wearer's torso by using fastening means that are usually of a belt or corset type.

The system of straps must be adjusted in length in order to ensure the correct adherence of the brace to the patient's spinal column and the right system of thrusts acting upon the affected anatomic regions.

The technical problem encountered in these solutions regards the connection between the vertically elongate rigid frame, which follows the shape of the spinal column, and the straps enabling it to be retained on the back, resulting in a certain obstacle to the movements the patient can make.

More in general these types of braces are poorly suited to being used for a postural correction, but are rather suited for a more complex action of the proprioceptive type which acts on the dorsal structure.

In the orthopaedic-health sector and in the sector of clothing, including sportswear, in addition to orthoses of the corset type, there are also known some technical-orthopaedic solutions which are based on the manufacture of garments whose effect on the wearer's body is to exert stimuli with diversified tensions which are capable of improving posture.

Among these solutions there are posture shirts produced by cutting and sewing different pieces making up the shirt, or else by means of a system of a seamless type, which provides for the development of a model using software for the various components of the item of clothing, which are in any case joined together.

The use of the seamless technique, which defines a garment without sewn stitches, is increasingly taking hold both in the technical sports sector and in the fashion industry, and entails the use of knitting machines that almost completely eliminate the need to produce and then sew together fabric parts.

Fitting into this sector are the shirts obtained by producing a first base layer of threads associated by weaving joined to areas of tension that are diversified by means of second threads of a larger size and greater elastic resistance, suitable for creating in those areas a higher tension which is transformed into traction capable of stimulating the muscles in the area responsible for postural control.

Such posture shirts are increasingly used both in the sports sector and in the orthopaedic sector, but to date there are no known solutions that envisage the combination thereof with rigid or semi-rigid supports placed along the spinal column, which would be desirable if one wished to correct more serious postural disorders without relying on classic orthoses, which are highly cumbersome, difficult to use and impractical in the cases of use by elderly or disabled persons.

### DESCRIPTION OF THE INVENTION

The present invention aims to provide a posture shirt made with a conformation that is adapted to include reinforcement elements for the spinal column, thereby creating a condition capable of eliminating or at least reducing the drawbacks highlighted above.

The invention aims in particular to provide a posture shirt comprising a housing in which a bar is inserted, i.e. a rigid vertical dorsal structure that follows the line of the spinal column, remaining adherent by virtue of a non-elastic strip secured at the waist.

This is obtained by means of a posture shirt comprising stiffening elements for the spinal column, whose features are described in the main claim.

The dependent claims of the solution in question outline advantageous embodiments of the invention.

The main advantages of this solution regard the fact that the posture shirt comprises a dorsal stiffening element that considerably improves its efficiency, since a part that is essential for the torso, represented precisely by the spinal column, remains protected and supported.

A further advantage offered by the posture shirt according to the invention is that of offering wearers with a postural deficiency the possibility of using the combination thereof with a rigid support for the torso that is extremely efficient and comfortable.

### ILLUSTRATION OF THE DRAWINGS

Other features and advantages of the invention will become more apparent on reading the following description of an embodiment of the invention, provided by way of nonlimiting example with the aid of the drawings illustrated in the attached figures, in which:
- figure 1 represents a schematic view of the posture shirt according to the invention in a condition of normal use with the upper torso erect;
- figure 2 illustrates a schematic view showing the same posture shirt according to the invention in a condition of use with the upper torso bent forward.

### DESCRIPTION OF AN EMBODIMENT OF THE INVENTION

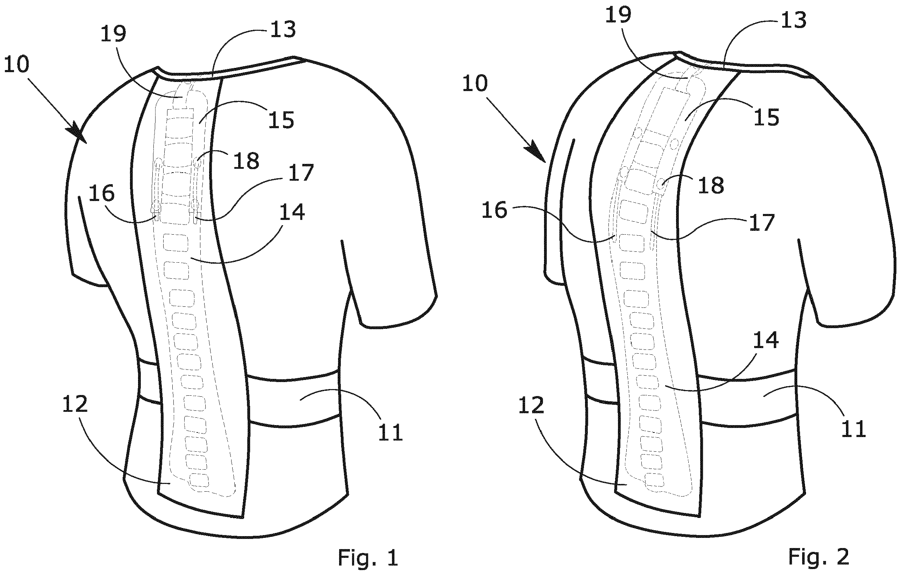

Making reference to the appended figures, and initially in particular to figure 1, 10 generally indicates a shirt wearable by a user who intends to correct or alleviate postural problems or usable in the orthopaedic sector for controlled retention and corrective anatomic support of the spinal column.

According to some embodiments, the posture shirt 10 is preferably, albeit not necessarily, produced as an integral item of clothing with a three-dimensional structure (3D), possibly seamless, and designed to control posture, the shirt being obtained by weaving with areas with diversified traction, intended, according to the zones, to ensure stimulation of the muscles responsible for controlling posture in order to restore a correct attitude in subjects affected by disabling pathologies such as, for example, hyperkyphotic dysmorphisms.

According to a preferred embodiment of the invention the shirt 10 comprises a substantially rigid strip 11, fixable to the wearer's waist, and a pocket 12 formed in the dorsal zone of the shirt, i.e. in the median vertical zone located along the spinal column.

According to the embodiment represented in figure 1, the pocket 12 is disposed between the lower lumbar zone and the upper end of the shirt in the cervical zone of the collar 13.

In the upper zone, the pocket 12 has an opening for the introduction of a reinforcement element 14, as described below.

The distinctive feature of the invention in fact lies in the fact of introducing inside the pocket 12 a rigid or semi-rigid reinforcement element or bar 14 which represents a dorsal support in that it has an elongated vertical extent, having a conformation that corresponds to a spinal column, allowing it to follow the shape and rest upon the latter and to be held in place thanks to the traction of the rigid strip fixable to the wearer's waist.

Said reinforcement element or bar 14 terminates at the top with a slide 15 that is slidable vertically along the bar, that is, along the median axis thereof.

More in particular, according to the embodiment represented in figure 1, the bar 14 comprises at the top two parallel slots 16 and 17 in which respective pins integral with the slide 15 operate, so that the latter may slide from a lowered position to a raised position relative to the bar 14.

The upper end of the slide 15 is associated with a strap 19 which connects the slide to the edge of the collar 13.

The strip 11 is, as noted, of a non-elastic type, as it must be able to perform the dual function of retaining the bar 14, maintaining the correct positioning thereof inside the pocket 12, and of maintaining the necessary adherence of the bar to the wearer's back when he or she bends forward.

From an operational standpoint, the posture shirt 10 according to the invention provided with a semi-rigid reinforcement element 14 placed inside the pocket 12 enables the slide 15 to facilitate the movement thereof by moving upwards relative to the bar 14 when the wearer bends forward, as represented in figure 2, preventing the bar from shifting and ensuring the recall effect given by the bar itself.

In fact, during the traction exerted as a result of the wearer bending forward, the slide is drawn upward by the strap 19 fixed onto collar of the shirt, whereas during the return to the erect position it is the shirt 10 itself, which is preferably of the specifically elasticised type, that brings the slide back into the initial position.

As it is possible to note, the posture shirt according to the invention makes it possible to obtain the advantageous results for which it was designed, in particular the result obtained from the combination with the dorsal stiffening element 14, which considerably improves its efficiency, since a part that is essential for the torso, represented precisely by the spinal column, remains protected and supported.

The invention has been described above with reference to a preferred embodiment thereof. However, it is clear that the invention is susceptible of numerous variants falling within the scope thereof, within the framework of technical equivalences.

## Claims

1. A posture shirt (10) designed for controlling posture, which is obtained by weaving with areas with diversified traction, intended, according to the zones, to ensure stimulation of the muscles responsible for controlling posture in order to restore a correct attitude in subjects affected by disabling pathologies such as hyperkyphotic dysmorphisms, said shirt (10) comprising at the top a collar (13), **characterised in that** it comprises a substantially rigid strip (11), fixable to the wearer's waist, associated with a pocket (12) formed in the dorsal zone of the shirt located along the spinal column, wherein a reinforcement element or bar (14) is insertable, the reinforcement element or bar (14) representing a dorsal support terminating at the top with a slide (15) that is slidable vertically along the median axis of the bar.

2. The posture shirt (10) according to claim 1, **characterised in that** an upper zone of the slide (15) is associated with a strap (19) which connects the end of the slide (15) to the edge of the collar (13).

3. The posture shirt (10) according to one of the preceding claims, **characterised in that** said pocket (12) is disposed between the lower lumbar zone and the upper zone of the shirt located in the cervical zone of the collar (13).

4. The posture shirt (10) according to claim 3, **characterised in that** in said upper zone the pocket (12) has an opening for the introduction of said reinforcement element or bar (14).

5. The posture shirt (10) according to one of the preceding claims, **characterised in that** said reinforcement element or bar (14) is of a rigid or semi-rigid type and represents a dorsal support **in that** it has an elongated vertical extent, having a conformation that corresponds to a spinal column, allowing it to follow the shape and rest upon the latter and to be held in place thanks to the traction of said rigid strip (11) fixable to the wearer's waist.

6. The posture shirt (10) according to one of the preceding claims, **characterised in that** said reinforcement element or bar (14) comprises at the top two parallel slots (16, 17) in which respective pins integral with the slide (15) operate, so that the latter may slide from a lowered position to a raised position relative to the bar (14).

7. The posture shirt (10) according to one of the preceding claims, **characterised in that** said strip (11) is of a non-elastic type, as it must be able to retain the bar (14), maintaining the correct positioning thereof inside the pocket (12), and to maintain the necessary adherence of the bar to the wearer's back when he or she bends forward.

## Patentansprüche

1. Körperhaltungshemd (10), das zum Kontrollieren der Körperhaltung gestaltet ist, das erhalten wird durch Weben mit Flächen mit diversifiziertem Zug, dafür vorgesehen, entsprechend den Bereichen, eine Stimulierung der Muskeln, die für das Kontrollieren der Körperhaltung verantwortlich sind, sicherzustellen, um eine richtige Haltung bei Personen wiederherzustellen, die durch beeinträchtigende Krankheitsbilder, wie beispielsweise hyperkyphotischer Dysmorphie, betroffen sind, wobei das Hemd (10) an dem Oberteil einen Kragen (13) umfasst, **dadurch gekennzeichnet, dass** es einen im Wesentlichen starren Streifen (11) umfasst, der an der Taille des Trägers befestigt werden kann, verknüpft mit einer Tasche (12), die in dem dorsalen Bereich des Hemdes geformt ist, worin ein Verstärkungselement oder eine Schiene (14) eingesetzt werden kann, wobei das Verstärkungselement oder die Schiene (14) eine dorsale Stütze darstellt, die an dem Oberteil mit einem Gleitstück (15) endet, das entlang der medianen Achse der Schiene in Vertikalrichtung verschiebbar ist.

2. Körperhaltungshemd (10) nach Anspruch 1, **dadurch gekennzeichnet, dass** ein oberer Bereich des Gleitstücks (15) mit einem Gurt (19) verknüpft ist, der das Ende des Gleitstücks (15) mit der Kante des Kragens (13) verbindet.

3. Körperhaltungshemd (10) nach einem der vorhergehenden Ansprüche, **dadurch gekennzeichnet, dass** die Tasche (12) zwischen dem Lendenbereich und dem oberen Bereich des Hemdes, der sich in dem Halsbereich des Kragens (13) befindet, angeordnet ist.

4. Körperhaltungshemd (10) nach Anspruch 3, **dadurch gekennzeichnet, dass** die Tasche (12) in dem oberen Bereich eine Öffnung für das Einführen des Verstärkungselements oder der Schiene (14) aufweist.

5. Körperhaltungshemd (10) nach einem der vorhergehenden Ansprüche, **dadurch gekennzeichnet, dass** das Verstärkungselement oder die Schiene (14) von einer starren oder halbstarren Art ist und insofern eine dorsale Stütze darstellt, als es/sie eine langgestreckte vertikale Ausdehnung aufweist, wobei es/sie eine Gestaltung aufweist, die einer Wirbelsäule entspricht, was ermöglicht, dass es/sie der Form folgt und an der letzteren anliegt und dank des Zuges des starren Streifens (11), der an der Taille des Trägers befestigt werden kann, am Platz gehalten wird.

6. Körperhaltungshemd (10) nach einem der vorhergehenden Ansprüche, **dadurch gekennzeichnet, dass** das Verstärkungselement oder die Schiene (14) an dem Oberteil zwei parallele Schlitze (16, 17) umfasst, in denen jeweilige Stifte, integral mit dem Gleitstück (15), arbeiten, so dass das letztere von einer abgesenkten Stellung zu einer erhöhten Stellung im Verhältnis zu der Schiene (14) gleiten kann.

7. Körperhaltungshemd (10) nach einem der vorhergehenden Ansprüche, **dadurch gekennzeichnet, dass** der Streifen (11) von einer nicht-elastischen Art ist, da er dazu in der Lage sein muss, die Schiene (14) festzuhalten, die richtige Positionierung derselben innerhalb der Tasche (12) aufrechtzuerhalten und die notwendige Anhaftung der Schiene am Rücken des Trägers, wenn er oder sie sich nach vorn beugt, aufrechtzuerhalten.

## Revendications

1. T-shirt correcteur de posture (10) conçu pour le contrôle de la posture, obtenu par tissage avec des zones de traction diversifiée, conçues, selon les zones, pour assurer la stimulation des muscles responsables du contrôle de la posture pour rendre un maintien correct à des sujets atteints de pathologies invalidantes telles que les dysmorphies hypercyphotiques, ledit T-shirt (10) comprenant en partie haute un col (13), **caractérisé en ce qu'**il comprend une bande sensiblement rigide (11) pouvant être fixée à la taille de la personne portant le T-shirt, associée à une poche (12) formée dans la zone dorsale du T-shirt située le long de la colonne vertébrale, dans laquelle peut être inséré un élément ou une barre de renfort (14), l'élément ou la barre de renfort (14) constituant un support dorsal se terminant en partie haute par une glissière (15) pouvant coulisser verticalement le long de l'axe médian de la barre.

2. T-shirt correcteur de posture (10) selon la revendication 1, **caractérisé en ce qu'**une zone supérieure de la glissière (15) est associée à une sangle (19) qui relie l'extrémité de la glissière (15) au bord du col (13).

3. T-shirt correcteur de posture (10) selon l'une des revendications précédentes, **caractérisé en ce que** ladite poche (12) est disposée entre la zone lombaire inférieure et la zone supérieure du T-shirt située dans la zone cervicale du col (13).

4. T-shirt correcteur de posture (10) selon la revendication 3, **caractérisé en ce que**, dans ladite zone supérieure, la poche (12) présente une ouverture destinée à l'introduction dudit élément ou barre de renfort (14).

5. T-shirt correcteur de posture (10) selon l'une des revendications précédentes, **caractérisé en ce que** ledit élément ou barre de renfort (14) est de type rigide ou semi-rigide et constitue un support dorsal **en ce qu'**il présente une extension verticale allongée, présentant une conformation qui correspond à une colonne vertébrale, lui permettant d'épouser la forme de et de reposer sur celle-ci et d'être maintenu en place grâce à la traction de ladite bande rigide (11) pouvant être fixée à la taille de la personne portant le T-shirt.

6. T-shirt correcteur de posture (10) selon l'une des revendications précédentes, **caractérisé en ce que** ledit élément ou barre de renfort (14) comprend en partie haute deux fentes parallèles (16, 17) dans lesquelles opèrent des broches respectives solidaires de la glissière (15), de sorte que celle-ci peut coulisser d'une position basse à une position haute par rapport à la barre (14).

7. T-shirt correcteur de posture (10) selon l'une des revendications précédentes, **caractérisé en ce que** ladite bande (11) est de type non-élastique, dans la mesure où elle doit être capable de retenir la barre (14), en maintenant son positionnement correct à l'intérieur de la poche (12), et de maintenir l'adhérence nécessaire de la barre au dos de la personne portant le T-shirt quand celle-ci se penche en avant.
